# EUROPEAN PATENT SPECIFICATION

(11) **EP 2 560 701 B1**
(45) Date of publication and mention of the grant of the patent: **01.11.2017**
(21) Application number: 11723650.5
(22) Date of filing: 20.04.2011
(51) Int. Cl.: A61M 1/16, A61M 1/28

(54) **FILTERING AND PUMPING APPARATUS FOR MEDICAL USE**
FILTRIER- UND PUMPGERÄT FÜR MEDIZINISCHE ZWECKE
APPAREIL DE FILTRATION ET DE POMPAGE POUR UNE UTILISATION MÉDICALE

(30) Priority: 21.04.2010 IT MO20100119
(43) Date of publication of application: 27.02.2013
(73) Proprietor: Rand S.r.l., 41036 Medolla (MO) (IT)
(72) Inventor: GALAVOTTI, Daniele, I-41037 Mirandola (MO) (IT); RONCO, Claudio, I-36100 Vicenza (VI) (IT)
(74) Representative: Bergamini, Silvio
(86) International application number: PCT/IB2011/051718
(87) International publication number: WO 2011/132154

(56) References cited:
- WO-A1-2008/082528
- WO-A1-2009/051669
- US-A- 4 083 777
- US-A1- 2006 241 543
- US-A1- 2009 120 864

## Description

### Field of the invention

The present invention relates to a filtering and pumping apparatus for medical use, particularly for hemofiltration, which is particularly adapted to be carried by a patient during performance of his/her routine daily activities, or to be inserted in a garment worn by the patient.

### Background art

The prior art comprises devices for filtering and pumping patient's blood, which are used in extracorporeal hemofiltration techniques, and consist of a filter element through which the patient's blood to be purified is caused to pass, and a pump for maintaining active blood circulation through the filter.

At present, these two components, i.e. the filter and the pump, are functionally connected by special tube lines but are structurally separated, although they are mounted in the same hemofiltration machine that is adapted to be connected with a patient.

The reason for this is that the materials that form these two apparatus are very different, namely the filter is made of a biocompatible, disposable plastic material, whereas the pump is mostly made of metal.

In prior art filtering apparatus, the pump is typically a peristaltic pump, i.e. a pump comprising a motor that rotates an eccentric member, which alternately presses an elastic deformable pipe located near the eccentric member, thereby generating a pulsating thrust.

A "wearable dialysis methods and devices" is known from US 2009/0120864.

According to this patent a portable continuous dialysis system is configured as a wearable belt in fluid communication with a separate portable unite in the form of an easy to carry bag-pack or case, or a fanny pack wearable around the shoulder.

In one embodiment, the wearable belt unit comprises a dialyzer and a pump, such as a dual pulsatile pump, while the portable unit comprises a dialysate regeneration system and a waste collection bag.
In another embodiment, the wearable belt unit comprises a manifold for dialysate circuit. The placement of components can be varied between the portable unit and the wearable belt unit, depending upon factors such as comparative weight and size of the belt and portable units, the easy of operation of the dialysis system by the patient, the overall length of the tubing system and the safety of operation of the overall system.

The above described prior art suffers from certain drawbacks.

One drawback is that the separation between the filter and the pump requires the patients to use an external machine that comprises both, to perform hemofiltration cycles, which forces them to stop their daily activities during the time in which extracorporeal treatment is performed.

Another drawback is that the diversity of the materials used to make the filter and the pump makes the processes of making and assembling hemofiltration machines more problematic, because these materials may have incompatible features, e.g. in terms of elasticity, deformation, mechanical strength, and cause assembly problems, as well as problematic compatibility with the patients' bodies.

A further drawback is that, for hemofiltration treatment, prior art machines require a considerable volume of blood to be drawn from the patients to fill the whole hemofiltration circuit and the conduits that connect the external machine to the patient, the latter being sometimes located at a few meters from the machine, thus requiring the use of long conduits to be filled with blood.

Such blood withdrawal from the patients requires a corresponding volume recovery in the organism, which has to be therefore obtained by infusing replacement saline solutions into the patients.

Another drawback is that the manufacture of prior art filtering apparatus has a high cost, due to the diversity of materials and the need for accurate assembly of the various parts.

A further drawback is that prior art filters have areas in which blood flow is slowed down, or even stopped, thereby creating stagnation and formation of undesired clots.

### Disclosure of the invention

It is an object of the present invention to improve the prior art.

Another object is to make a filtering and pumping apparatus for medical use that is very compact, and can be carried by a patient during performance of his/her daily activities, or possibly associated to a garment or a clothing accessory, and that can be made from biocompatible and homogeneous plastic materials, to be entirely disposable.

Yet another object of the invention is to provide a filtering and pumping apparatus for medical use that allows easy assembly and whose parts have no different elastic and mechanical features.

A further object of the invention is to provide a filtering and pumping apparatus for medical use that affords homogeneous hemofiltration, and prevents clot formation and blood stagnation.

In one aspect, the invention relates to a filtering and pumping apparatus for medical use as defined in claim 1.

Therefore, the invention provides the following advantages:
providing a very compact filtration apparatus, that can be carried by a patient during performance of routine daily activities;
providing accurate and constant hemofiltration with no clot formation or blood stagnation;
allowing hemofiltration treatment with little blood withdrawal from the patients;
customizing the filtering and pumping apparatus for medical use according to patients' requirement, providing simplified, low-cost embodiments that do not involve the use of a replacement fluid or more complete embodiments in which a replacement fluid can be also used, but in small amounts;
making a filtering and pumping apparatus for medical use that can be inserted in a garment.

### Brief description of the drawings

Further features and advantages of the invention will be more readily apparent upon reading of the detailed description of a few preferred not exclusive embodiments of a filtering and pumping apparatus for medical use, which are shown as a not limiting example with the help of the annexed figures, in which:
FIG. 1 is a partially longitudinal section schematic phantom view of a filtering and pumping apparatus for medical use according to a first embodiment of the invention;
FIG. 2 is a cross-sectional view of the filtering and pumping apparatus for medical use of Fig. 1, as taken along a plane *II - II;*
FIG. 3 is a partially longitudinal section schematic phantom view of a filtering and pumping apparatus for medical use according to a second embodiment of the invention;
FIG. 4 is a cross-sectional view of the filtering and pumping apparatus for medical use of Fig. 3, as taken along a plane *IV - IV;*
FIG. 5 is a partially longitudinal section schematic phantom view of a filtering and pumping apparatus for medical use according to a third embodiment of the invention;
FIG. 6 is a cross-sectional view of the filtering and pumping apparatus for medical use of Fig. 5, as taken along a plane *VI - VI;*

### Detailed description of one preferred embodiment

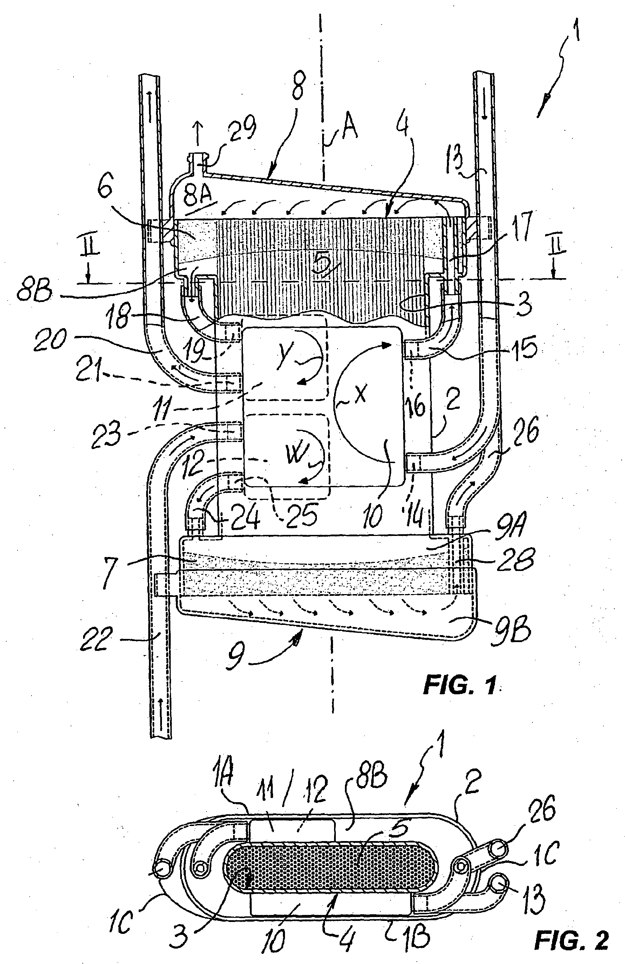

Referring to Fig. 1, numeral 1 designates a filtering and pumping apparatus for medical use, which comprises a flat and very compact container body 2.

The container body 2 or, in short, body 2, has a longitudinal axis "A" and defines therein a chamber 3 that holds a filter unit 4 comprising a plurality of hollow fibers 5 arranged in a bunch and parallel to the longitudinal axis "A".

The hollow fibers 5 are made from a porous material, that allows selective passage of solutes or materials in water solutions and retains molecularly larger particles, as explained below.

The hollow fibers 5 have their respective corresponding open ends secured in two lock elements 6 and 7, which are known in the art as "pottings" and are made of plastic.

As shown in the figures, two respective extension portions of the chamber 3 are defined in the body 2, which widen out with respect to the chamber and have a hollow interior.

These portions receive the corresponding ends of the hollow fibers 5, said portions being an inlet portion 8 and an outlet portion 9 respectively.

The two lock elements 6 and 7 are sealably held within these portions 8 and 9 and divide each of them into two half-portions 8A, 8B and 9A, 9B respectively.

As mentioned above, the body 2 is compact and has a very flat shape, as shown in Fig. 2, and defines two parallel and opposed flat faces 1A and 1B, connected together by rounded end surfaces 1C: this allows the body 2 to be inserted in a garment designed to be worn by a patient, and considerable reduces discomfort thereof.

The outer wall of the chamber 3 has, associated therewith, a first pump 10, whose dimensions are contained within the plan projection of the body 2, a second pump 11 and a third pump 12, which also have such dimensions as to be contained in the plan projection of the body 2, i.e. the plan projection of the drawings, with respect to a viewer.

The filtering and pumping apparatus 1, in short apparatus 1, also comprises a first conduit 13 that connects an inlet 14 of the first pump 10 and a second conduit 15 that connects an outlet 16 of the first pump 10 with a passage 17 formed in the lock element 6, which extends therethrough and opens into the half-portion 8A.

A third conduit 18 connects an outlet 19 of the half-portion 8B with an inlet of the second pump 11, whereas a fourth conduit 20 connects an outlet 21 of the second pump 11 with the outside.

An additional fifth conduit 22 connects an inlet 23 of the third pump 12 with the outside and a sixth conduit 24 connects an outlet 25 of the third pump 12 with the half-portion 9A.

Finally, a seventh conduit 26 connects the half-portion 9B with the outside, through an additional passage 28 that extends through the lock element 7.

According to the purification technique selected by the physician and the fluidic flow diagram derived therefrom, in an alternative embodiment of the filtering and pumping apparatus 1 the sixth conduit 24 may also directly flow into the seventh conduit 26, so that the replacement fluid is directly re-infused into the return line to the patient without interacting with the blood in the filter.

An air exhaust port 29 is also formed in the half-portion 8A, for exhausting any air in the device to the atmosphere.

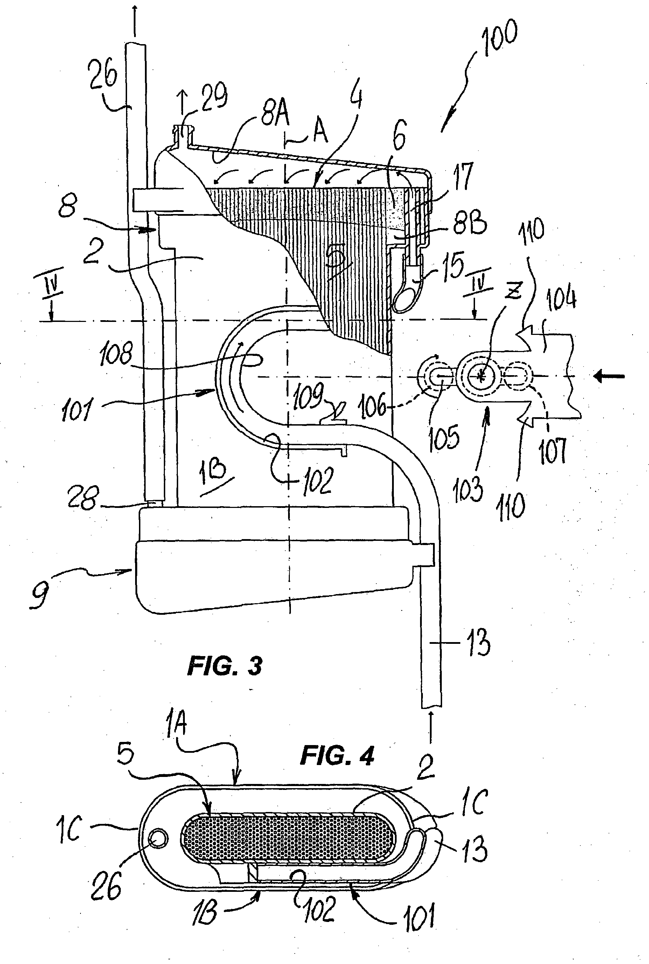

Referring to Figures 3 and 4, there is shown a second embodiment of the filtering and pumping apparatus of the invention, referenced 100.

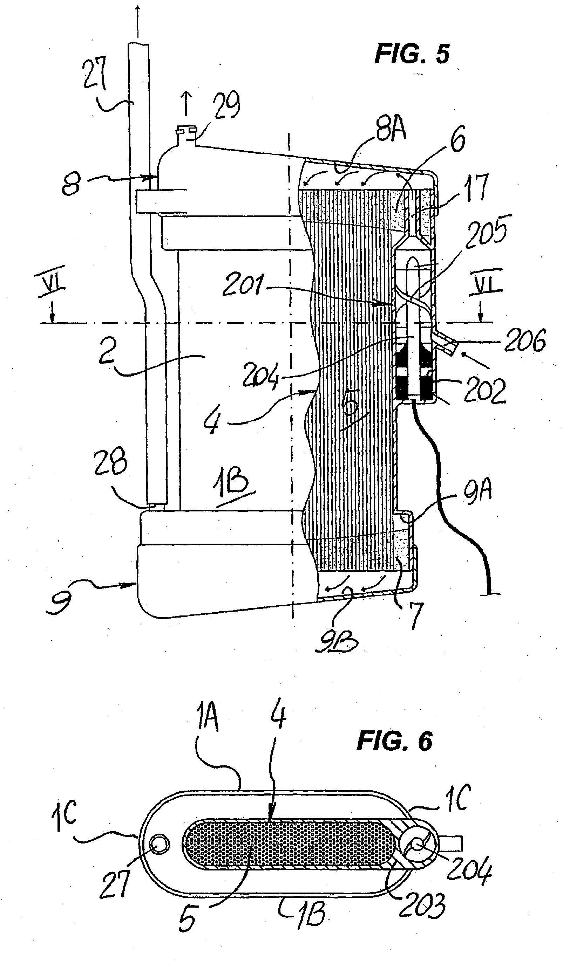

In these figures, like in Figures 5 and 6 as described below, reference numerals for elements shared by all the embodiments of the filtering and pumping apparatus, are maintained the same.

As shown in Fig. 3, the filtering and pumping apparatus 100 comprises a raised guide 101 that forms a semicircular groove 102 for receiving a section of the first conduit 13 through which a fluid designed to undergo hemofiltration is fed.

As shown in the figure, the semicircular groove 102 is formed on one of the flat faces of the body 2, namely the face 1B but if required it might also be formed on the opposite face 1A.

The section of the first conduit 13 forms a semicircular or substantially semicircular loop 108 which can receive in its cavity an active end 103 of a peristaltic pump 104, not entirely visible but known to the skilled person.

As shown in Fig. 3, the active end 103 supports a rotor 105 which is driven by a motor unit, not shown, to rotate about an axis of rotation "Z" which is substantially perpendicular to the face 1B.

The rotor 105 in turn has an elongate shape, symmetrical with respect to the axis "Z" and supports two rollers 106 and 107 at its opposite ends, which can freely rotate about their axes of rotation parallel to the axis "Z".

The active end 103 may be connected to the body 2 by hooks 109 and 110, arranged in such a way that, when this end is connected to the body 2, the rotation of the rotor 105 causes alternate contact between the rollers 106 and 107 and the curved section of the first section 13 and alternate compression thereof: such alternate compression generates the pumping action.

As the skilled person may appreciate, the hooks 109 and 110 are also designed to be disconnected from each other for removal of the end 103 from the body 2.

Once again in this second embodiment, it can be noted, particularly referring to Fig. 4, that no part of the filtering and pumping apparatus 100 projects out of the plan projection of the body 2.

Referring to Fig. 5, a further embodiment 200 of the filtering and pumping apparatus of the invention is shown.

In this embodiment, a pump 201 is arranged in a hollow housing 202 formed in projection 203 of the body 2, preferably, but without limitation, on one of the curved connecting surfaces between the flat faces 1A and 1B.

Thus, the hollow housing 202 substantially acts as a sleeve for a rotating shaft 204 which is driven by a motor "M" and has a peripheral raised helical profile 205, designed to push a fluid through the passage 17 with which the hollow housing communicates at one end.

The fluid that is designed to undergo hemofiltration is introduced into the pump 201 through an inlet 206 which is formed in one side of the hollow housing 202.

Namely, in all the embodiments of the filtering and pumping apparatus for medical use of the invention, any reference to a fluid that is designed to undergo hemifiltration is intended as patient's blood, whereas any reference to a replacement fluid shall be intended as a saline solution compatible with blood which may be enriched with electrolytes, particular nutritional elements or proteins, according to therapeutic needs.

The operation of the filtering and pumping apparatus for medical use is as follows: referring to Figures 1 and 2, the blood of a patient that is designed to undergo hemofiltration reaches the inlet 14 of the first pump 10 through the first conduit 13 that connects the circulatory system of the patient to the filtering and pumping apparatus of the invention.

The first pump 10 pushes blood toward the outlet 16 in the direction of the arrow "X" and from this outlet, through the second conduit 15, to the half-portion 8A of the inlet portion 8 of the body 2, through the passage 17 formed in the lock element 6.

From this half-portion 8A, blood enters the hollow fibers 5 and flows through them to the half-portion 8B of the outlet portion 9, wherefrom it is returned to the patient through the seventh conduit 26.

As it flows through the hollow fibers 5, which are made, as mentioned above, from a porous material, blood releases part of its water component, which is collected in the half-portion 8B of the inlet portion 8 and enters the second pump, through the third conduit 18, from which it is pushed outwards in the direction of arrow "Y" through the fourth conduit 20 fitted on the outlet 21.

In order to compensate for blood volume loss caused by separation of the water component, a saline solution is introduced through the fifth conduit 22 which feeds it into the third pump 12 through the inlet 23.

The third pump 12 pushes the saline solution into the half-portion 9A of the outlet portion 9 of the body 2, in the direction of arrow "W", through the outlet 25 and the sixth conduit 24.

From this half-portion 9A, the saline solution is pushed to flow over the hollow fibers 5 in countercurrent flow relative to blood flow in the latter.

The porosity of the material of which the hollow fibers 5, i.e. the filter unit 4, are made, allows the saline solution to flow through them and combine with blood, thereby substantially restoring its initial volume.

In the therapeutic scheme as described before, the possibility of modulating the speed of the pump 11 relative to that of the pump 12, determines the water volume, also known as water balance, that may be restored to the patient (which water balance may be an even balance or positive or negative, as required by the physician), and possibly enriched with particular substances of the replacement liquid, to achieve a higher therapeutic effect.

As an alternative to the above described therapeutic scheme, and according to a selected purification technique deriving from the fluidic flow diagram selected by the physician, in an additional embodiment of the filtering and pumping apparatus of the invention the conduit 24 may also directly open into the conduit 26, so that the replacement fluid can be directly re-infused on the return line to the patient, without interacting with the blood in the filter.

It shall be particularly noted that the filtering and pumping apparatus 1 has a flat shape with no part projecting out of such shape, which makes it particularly suitable for arrangement in a container or in a pocket of a garment.

Referring to the embodiment of the filtering and pumping apparatus as shown in Figures 3 and 4, it shall be noted that a single peristaltic pump 104 is provided; which may connected or disconnected to and from the body 2 as needed, using the hooks 109 and 110.

The rollers 106 and 107 of the rotatably driven rotor 105 alternately compress the section of the first conduit 13 that is formed of a plastic and elastic material and forms the loop 108, thereby generating the pumping effect.

The patient's blood is pushed by the pump 104 into the half-portion 8A through the second conduit 15 and the passage 17, and flows therefrom through the hollow fibers, to reach the half-portion 9B (not shown, but easily understood by the skilled person) and then is returned to the patient after filtration through the second conduit 26 after passing through the additional passage 28.

In this second variant embodiment of the filtering and pumping apparatus 100 of the invention, the second pump 11 and the third pump 12 are omitted, as filtration occurs by a slow flow, and the blood volume loss in the patient is negligible, or anyway will not cause any safety hazard.

The water solution that is filtered by the filtering and pumping apparatus 100 is collected in a container or a discharge bag that may be ergonomically integrated in the apparatus and worn by the patient, to collect a volume generated during 24 hours' use or at least have such a collection capacity as to limit the manual emptying operations to once or twice in a day.

Referring now to Figures 5 and 6, which show a third possible embodiment of the filtering and pumping apparatus 200, the blood of the patient is shown to be introduced into the hollow housing 202 through the inlet port 206, wherefrom it is pushed by the helical profile 205 that rotates with the rotating shaft 204 driven by the motor M into the half-portion 8A of the inlet portion 8 of the body 2, after flowing through the passage 17 like in the previous embodiments.

Therefore, the filtration cycle is exactly as described in the previous embodiment of the filtering and pumping apparatus 100.

The invention was found to fulfill the intended objects.

The invention so conceived is susceptible to a number of changes and variants within the inventive concept.

Furthermore, all the details may be replaced by other technically equivalent parts. In practice, any materials, shapes and sizes may be used as needed, without departure from the scope of the following claims.

## Claims

1. A filtering and pumping apparatus (1; 100; 200) for medical use comprising:
- Feeding lines and removal lines (13, 15, 18, 20, 22, 24, 26) of a fluid to be pumped and filtered;
- A pumping unit (10, 11, 12) to pump said fluid along said lines (13, 15, 18, 20, 22, 24, 26);
- A filter unit (4) to filter said fluid;
said lines (13, 15, 18, 20, 22, 24, 26), said pumping unit (10, 11, 12), said filter unit (4) being all reciprocally associated in a single compact body (2) **characterized in that** said single compact body (2) comprises a flat body which has a longitudinal axis (A) and a longitudinal housing chamber (3) obtained therein and wherein a filter unit (4) is housed, said longitudinal housing chamber (3) having at least one outer projection projecting therefrom toward, but not over, one of two opposed flat outer faces (1A, 1B) defined by said flat body, so that said projection has dimensions contained within the plan projection of said flat body on a plane perpendicular to said longitudinal axis (A), within said projection said pumping unit (10, 11, 12) is housed.

2. An apparatus according to claim 1, wherein said pumping unit (10, 11, 12) and said filter unit (4) are made of biocompatible and substantially similar materials.

3. An apparatus according to claim 1, wherein said first face (1A) and second face (1B) are substantially planar and reciprocally parallel.

4. An apparatus according to claim 1, wherein said first face (1A) and second face (1B) are substantially concave and reciprocally parallel.

5. An apparatus according to claim 1 wherein said filter unit (4) comprises a filter comprising hollow filtering fibres (5) which are reciprocally joined and have respective first inlet ends and second outlet ends of said fluid which are open and which flow into a correspondent inlet portion (8) and outlet portion (9) of said chamber (3).

6. An apparatus according to claim 5, wherein said first inlet ends and said second outlet ends are joined together by joining means (6, 7).

7. An apparatus according to claim 6, wherein said joining means comprise respective blocks (6, 7) inside which said first inlet ends and said second outlet ends are blocked and which divide said inlet portions (8) and outlet portions (9) of said chamber (3) into first semi-portions (8A, 9A) defined upstream said of said blocks (6, 7) and second semi-portions (8B, 9B) defined downstream said blocks (6, 7).

8. An apparatus according to claims 1 and 7, wherein said pumping unit comprises at least a first fluid circulation pump (10) interposed between a feeding line (13) from the outside and a connecting line (15) with said first semi-portion (8A) of the inlet portion (8).

9. An apparatus according to anyone of preceding claims, wherein said pumping unit comprises a second circulation pump (11) designed to circulate in counter current a second fluid in respect of said first fluid, said second pump (11) being interposed between an outlet line (18) from said second semi-portion (8B) of said inlet portion (8) and a line (20) flowing away toward the outside.

10. An apparatus according to anyone of preceding claims, wherein said pumping unit comprises a third circulation pump (12) of a third fluid replacing the aqueous fraction of said first fluid.

11. An apparatus according to anyone of preceding claims, wherein said apparatus (1; 100; 200) can be associated to an item of clothing wearable by a patient.

## Patentansprüche

1. Filter- und Pumpenvorrichtung (1; 100; 200) zur medizinischen Verwendung, umfassend:
- Zulaufleitungen und Ablaufleitungen (13, 15, 18, 20, 22, 24, 26) eines Fluids, das gepumpt und gefiltert werden soll;
- eine Pumpeinheit (10, 11, 12), um das Fluid entlang der Leitungen (13, 15, 18, 20, 22, 24, 26) zu pumpen;
- eine Filtereinheit (4), um das Fluid zu filtern;
wobei die Leitungen (13, 15, 18, 20, 22, 24, 26), die Pumpeinheit (10, 11, 12), die Filtereinheit (4) alle wechselseitig in einem einzigen kompakten Körper (2) in Verbindung gebracht sind, **dadurch gekennzeichnet, dass** der einzige kompakte Körper (2) einen flachen Körper umfasst, der eine longitudinale Achse (A) und eine darin enthaltene longitudinale Gehäusekammer (3) hat, und in dem eine Filtereinheit (4) untergebracht ist, wobei die longitudinale Gehäusekammer (3) mindestens einen äußeren Vorsprung hat, der von dieser in Richtung, nicht jedoch darüber hinaus, einer von zwei entgegengesetzten flachen Außenflächen (1A, 1B), definiert durch den flachen Körper, hinausragt, so dass dieser Vorsprung Abmessungen hat, die innerhalb der Grundrissprojektion des flachen Körpers auf eine Fläche senkrecht zu der longitudinalen Achse (A) enthalten sind, wobei innerhalb dieser Projektion die Pumpeinheit (10, 11, 12) untergebracht ist.

2. Vorrichtung nach Anspruch 1, wobei die Pumpeinheit (10, 11, 12) und die Filtereinheit (4) aus biokompatiblen und im Wesentlichen ähnlichen Materialien gefertigt sind.

3. Vorrichtung nach Anspruch 1, wobei die erste Fläche (1A) und die zweite Fläche (1B) im Wesentlichen planar und wechselseitig parallel sind.

4. Vorrichtung nach Anspruch 1, wobei die erste Fläche (1A) und die zweite Fläche (1B) im Wesentlich konkav und wechselseitig parallel sind.

5. Vorrichtung nach Anspruch 1, wobei die Filtereinheit (4) einen Filter umfasst, der hohle Filterfasern (5) umfasst, die wechselseitig verbunden sind und entsprechende erste Einlassenden und zweite Auslassenden des Fluids haben, die offen sind, und die in einen entsprechenden Einlassabschnitt (8) und einen Auslassabschnitt (9) der Kammer (3) münden.

6. Vorrichtung nach Anspruch 5, wobei die ersten Einlassenden und die zweiten Auslassenden mittels Verbindungsmitteln (6, 7) miteinander verbunden sind.

7. Vorrichtung nach Anspruch 6, wobei die Verbindungsmittel entsprechende Blockierungen (6, 7) umfassen, in denen die ersten Einlassenden und die zweiten Auslassenden blockiert sind und die die Einlassabschnitte (8) und die Auslassabschnitte (9) der Kammer (3) in erste Halb-Abschnitte (8A, 9A), stromaufwärts der Blockierungen (6, 7) festgelegt, und zweite Halb-Abschnitte (8B, 9B), stromabwärts der Blockierungen (6, 7) festgelegt, unterteilen.

8. Vorrichtung nach Anspruch 1 und 7, wobei die Pumpeinheit mindestens eine erste Fluidumlaufpumpe (10) umfasst, die zwischen einer Zulaufleitung (13) von der Außenseite und einer Verbindungsleitung (15) mit dem ersten Halb-Abschnitt (8A) des Einlassabschnitts (8) eingefügt ist.

9. Vorrichtung nach einem der vorhergehenden Ansprüche, wobei die Pumpeinheit eine zweite Fluidumlaufpumpe (11) umfasst, konzipiert, um in einem Gegenstrom ein zweites Fluid in Bezug auf das erste Fluid umlaufen zu lassen, wobei die zweite Pumpe (11) zwischen einer Auslassleitung (18) aus dem zweiten Halb-Abschnitt (8B) des Einlassabschnitts (8) und einer Leitung (20), die in Richtung auf die Außenseite läuft, eingefügt ist.

10. Vorrichtung nach einem der vorhergehenden Ansprüche, wobei die Pumpeinheit eine dritte Fluidumlaufpumpe (12) eines dritten Fluids umfasst, die den wässrigen Anteil des ersten Fluids ersetzt.

11. Vorrichtung nach einem der vorhergehenden Ansprüche, wobei die Vorrichtung (1; 100; 200) mit einem von einem Patienten tragbaren Kleidungsgegenstand in Verbindung gebracht werden kann.

## Revendications

1. Appareil de filtration et de pompage (1 ; 100 ; 200) à usage médical comprenant :
- des conduites d'alimentation et des conduites d'évacuation (13, 15, 18, 20, 22, 24, 26) d'un fluide à pomper et à filtrer ;
- une unité de pompage (10, 11, 12) pour pomper ledit fluide le long desdites conduites (13, 15, 18, 20, 22, 24, 26) ;
- une unité de filtration (4) pour filtrer ledit fluide ;
lesdites conduites (13, 15, 18, 20, 22, 24, 26), ladite unité de pompage (10, 11, 12), ladite unité de filtration (4) étant toutes associées mutuellement dans un corps compact unique (2), **caractérisé en ce que** ledit corps compact unique (2) comprend un corps plat qui présente un axe longitudinal (A) et une chambre de logement longitudinale (3) ménagée en son sein et dans lequel une unité de filtration (4) est logée, ladite chambre de logement longitudinale (3) ayant au moins une projection extérieure se projetant depuis celle-ci en direction, mais pas au-dessus, d'une de deux faces extérieures plates opposées (1A, 1B) définies par ledit corps plat, de sorte que ladite projection présente des dimensions contenues à l'intérieur de la projection en plan dudit corps plat sur un plan perpendiculaire audit axe longitudinal (A), ladite unité de pompage (10, 11, 12) étant logée à l'intérieur de ladite projection.

2. Appareil selon la revendication 1, dans lequel ladite unité de pompage (10, 11, 12) et ladite unité de filtration (4) sont constituées de matériaux biocompatibles et sensiblement similaires.

3. Appareil selon la revendication 1, dans lequel lesdites première face (1A) et seconde face (1B) sont sensiblement planes et mutuellement parallèles.

4. Appareil selon la revendication 1, dans lequel lesdites première face (1A) et seconde face (1B) sont sensiblement concaves et mutuellement parallèles.

5. Appareil selon la revendication 1, dans lequel ladite unité de filtration (4) comprend un filtre comprenant des fibres de filtration creuses (5) qui sont reliées mutuellement et ont des premières extrémités d'entrée et des secondes extrémités de sortie respectives pour ledit fluide qui sont ouvertes et qui se déversent dans une partie d'entrée (8) et une partie de sortie (9) correspondantes de ladite chambre (3).

6. Appareil selon la revendication 5, dans lequel lesdites premières extrémités d'entrée et lesdites secondes extrémités de sortie sont reliées ensemble par des moyens de liaison (6, 7).

7. Appareil selon la revendication 6, dans lequel lesdits moyens de liaison comprennent des blocs respectifs (6, 7) à l'intérieur desquels lesdites premières extrémités d'entrée et lesdites secondes extrémités de sortie sont bloquées et qui divisent lesdites parties d'entrée (8) et parties de sortie (9) de ladite chambre (3) en des premières semi-parties (8A, 9A) définies en amont desdits blocs (6, 7) et en des secondes semi-parties (8B, 9B) définies en aval desdits blocs (6, 7).

8. Appareil selon les revendications 1 et 7, dans lequel ladite unité de pompage comprend au moins une première pompe de circulation de fluide (10) interposée entre une conduite d'alimentation (13) depuis l'extérieur et une conduite de connexion (15) avec ladite première semi-partie (8A) de la partie d'entrée (8).

9. Appareil selon l'une quelconque des revendications précédentes, dans lequel ladite unité de pompage comprend une deuxième pompe de circulation (11) conçue pour faire circuler à contre-courant un deuxième fluide par rapport audit premier fluide, ladite deuxième pompe (11) étant interposée entre une conduite de sortie (18) de ladite seconde semi-partie (8B) de ladite partie d'entrée (8) et une conduite (20) s'écoulant au loin vers l'extérieur.

10. Appareil selon l'une quelconque des revendications précédentes, dans lequel ladite unité de pompage comprend une troisième pompe de circulation (12) d'un troisième fluide remplaçant la fraction aqueuse dudit premier fluide.

11. Appareil selon l'une quelconque des revendications précédentes, dans lequel ledit appareil (1 ; 100 ; 200) peut être associé à une pièce vestimentaire pouvant être portée par un patient.
